# EUROPÄISCHE PATENTSCHRIFT

(11) **EP 2 404 632 B1**
(45) Veröffentlichungstag und Bekanntmachung des Hinweises auf die Patenterteilung: **21.11.2012**
(21) Anmeldenummer: 10007140.6
(22) Anmeldetag: 10.07.2010
(51) Int. Cl.: A61B 5/00, A61M 5/158

(54) **Insertionssystem für Nadeln**
Insertion system for needles
Système d'insertion pour des aiguilles

(43) Veröffentlichungstag der Anmeldung: 11.01.2012
(73) Patentinhaber: Roche Diagnostics GmbH, 68305 Mannheim (DE); F. Hoffmann-La Roche AG, 4070 Basel (CH)
(72) Erfinder: Deck, Frank, 67150 Niederkirchen (DE)
(74) Vertreter: Twelmeier Mommer & Partner

(56) Entgegenhaltungen:
- EP-A1- 1 698 279
- WO-A1-2008/133702
- WO-A1-2009/047512
- WO-A2-2009/024521
- US-A1- 2002 156 376
- US-A1- 2009 192 469

## Beschreibung

Die Erfindung geht aus von einem Insertionssystem mit den im Oberbegriff des Anspruchs 1 angegebenen Merkmalen. Ein solches Insertionssystem zum Insertieren eines Sensors oder einer Kanüle in Fettgewebe ist aus der WO 2009/047512 A1 bekannt.

Aus der WO 89/11820 ist System zur Insertion eines Sensors in fötale Kopfhaut bekannt. Bei diesem System wird eine halbkreisförmig gebogene Insertionsnadel mit einer Rotationsbewegung in die Haut eingestochen, so dass die Nadelspitze - ähnlich wie bei einer chirurgischen Naht - zu Beginn der Stechbewegung in der Haut eine Eintrittsöffnung erzeugt und am Ende der Stechbewegung in der Haut eine Austrittsöffnung erzeugt, aus der die Nadelspitze austritt.

Aus der US 2002/0156376 A1 ist ein Biopsiesystem bekannt, mit dem eine Nadel von einem mehrer Gelenke aufweisenden Arm geführt wird. Die Bewegung der Nadel wird dabei unter Berücksichtung von Ultraschallbildern gesteuert.

Aus der EP 1 698 279 A1 ist ein Insertionssystem zum Insertieren von Sensoren oder Kanülen bekannt, bei dem ein Einstechteil über eine Schenkelfeder auf einer in das Gehäuse integrierten Kurvenbahn in die Haut eingeführt und nach erfolgter Einführung in das Gehäuse zurückgezogen.

Sensoren zur Messung von Analytkonzentrationen in-vivo, beispielsweise der Glucosekonzentration, werden in unter der Haut liegendes Fettgewebe eines Patienten insertiert, indem eine Nadel in das Fettgewebe eingestochen wird. Hierfür gebräuchliche Insertionsnadeln sind in der Regel als Hohlnadeln oder V-förmige Rinnen ausgebildet, in denen ein Sensor liegt, beispielsweise ein Elektrodensystem für elektrochemische Messungen. Nach dem Einstich wird die Insertionsnadel aus dem Körpergewebe herausgezogen, wobei der Sensor in der erzeugten Stichwunde verbleibt.

In gleicher Weise werden Kanülen in Fettgewebe insertiert, beispielsweise zur Infusion von Insulin oder anderen medizinischen Wirkstoffen.

Insertionsvorrichtungen bestehen häufig aus einer Basiseinheit, die auf den Körper eines Patienten aufgeklebt wird, und einem Stechgerät, das für eine Insertion an die Basiseinheit angekoppelt und anschließend wieder abgenommen wird. Daneben sind auch Insertionsvorrichtungen bekannt, die nur aus einem Stechgerät bestehen.

Insertionssysteme werden häufig von Patienten selbst bedient, beispielsweise um Sensoren zur Messung der Glucosekonzentration zu insertieren. Bei der Entwicklung von Insertionssystemen ist es deshalb ein ständiges Ziel, dass diese möglichst einfach und sicher bedient werden können, Sensoren zuverlässig in Fettgewebe verankern und präzise Messungen ermöglichen. Zudem soll der mit dem Einstich einer Insertionsnadel verbundene Schmerz möglichst weitgehend minimiert werden.

Diese Aufgabe wird durch ein Insertionssystem mit den im Anspruch 1 angegebenen Merkmalen gelöst. Vorteilhafte Weiterbildungen der Erfindung sind Gegenstand von Unteransprüchen.

Der Stechmechanismus eines erfindungsgemäßen Insertionssystems bewegt den Halter der Insertionsnadel bei einem Stich zunächst linear in einer ersten Vorschubrichtung und ändert während des Stichs die Vorschubrichtung so, dass die Insertionsnadel am Ende des Stichs schräg oder senkrecht zu der ersten Vorschubrichtung ausgerichtet ist. Auf diese Weise können die folgenden Vorteile erreicht werden:
- Die Insertionsnadel kann mit einem steilen Winkel oder sogar senkrecht auf die Hautoberfläche auftreffen. Je steiler der Auftreffwinkel ist, desto besser kann die Insertionsnadel schmerzarm in unter der Haut liegendes Fettgewebe eindringen. Bei einem flachen Auftreffwinkel besteht nämlich die Gefahr, dass die Hautoberfläche der auftreffenden Nadel ausweicht und verschoben wird, was zusätzliche Schmerzen verursacht.
- Nach dem Durchstoßen der Hautoberfläche kann die Insertionsnadel bei einem erfindungsgemäßen Insertionssystem mit einer geänderten Vorschubrichtung schräg in das Unterhautfettgewebe geschoben werden. Dadurch lässt sich erreichen, dass der Sensor trotz einer kleinen Eindringtiefe der Insertionsnadel auf einer großen Länge von Fettgewebe umgeben ist. Durch eine reduzierte Eindringtiefe wird vorteilhaft die Gefahr einer Verletzung von unter dem Fettgewebe liegenden Muskeln reduziert und die für Messungen zur Verfügung stehende Länge des Sensors erhöht.

Die Erfindung ist insbesondere zur Insertion von Sensoren zur Messung von Analytkonzentrationen, beispielsweise der Konzentration von Glucose, Cholesterin oder Laktat geeignet.

Erfindungsgemäß deutet die Insertionsnadel am Ende der Stichbewegung in eine Richtung die schräg oder senkrecht zu der Richtung ist, in welche die Insertionsnadel bei Beginn der Stichbewegung deutet. Bevorzugt ist die Insertionsnadel gerade. Es kann aber auch eine gebogene Insertionsnadel verwendet werden. Unabhängig von ihrer Form ist eine Insertionsnadel in die Richtung ausgerichtet, in welche ihre Spitze deutet.

Eine erfindungsgemäße Stichbewegung kann beispielsweise erzeugt werden, indem der Insertionsnadelhalter des Insertionsgeräts bei einem Stich eine Bewegung durchführt, die einen Bogen enthält. Durch einen Bogen kann die Vorschubrichtung der Insertionsnadel geändert werden, so dass die Insertionsnadel am Ende des Stichs schräg oder senkrecht zu ihrer Orientierung bei Beginn eines Stichs ausgerichtet ist. Eine Änderung der Vorschubrichtung der Insertionsnadel kann im Anschluss an eine erste Bewegungsphase aber beispielsweise auch durch eine lineare Bewegung des Insertionsnadelhalters quer oder schräg zu der ursprünglichen Orientierung der Insertionsnadel, d.h. der Orientierung bei Beginn der Stechbewegung, erreicht werden.

Die Orientierung der Insertionsnadel bei Beginn der Stechbewegung stimmt bevorzugt mit der Vorschubrichtung bei Beginn der Stechbewegung überein.

Besonders vorteilhaft lässt sich eine erfindungsgemäße Stechbewegung verwirklichen, indem der Insertionsnadelhalter bei einem Stich eine Bewegung ausführt, die in einer Anfangsphase eine lineare Vorschubbewegung in einer ersten Vorschubrichtung und in einer Endphase eine lineare Vorschubbewegung in einer zweiten Vorschubrichtung ist, die schräg oder senkrecht zu der ersten Vorschubrichtung orientiert ist. Zwischen diesen beiden linearen Bewegungsabschnitten des Insertionsnadelhalters kann beispielsweise eine bogenförmige Bewegung des Insertionsnadelhalters oder eine Querbewegung des Insertionsnadelhalters erfolgen, um die Vorschubrichtung zu ändern.

Der Stechmechanismus zieht den Halter nach einer ersten Vorschubbewegung um einen Teil der bei der ersten Vorschubbewegung zurückgelegten Distanz zurück und bewirkt später eine zweite Vorschubbewegung. Die Haut kann nämlich von einer auftreffenden Insertionsnadel zunächst eingedellt werden, bevor die Insertionsnadel die Haut durchstößt. Es ist deshalb in der Regel vorteilhaft, die Länge der ersten Vorschubbewegung etwas größer zu wählen als für das Durchstechen der Haut bei idealen Bedingungen an sich erforderlich wäre. Nach der ersten Vorschubbewegung kann die Nadel dann im Fettgewebe etwas zurückgezogen werden und später die zweite Vorschubbewegung anschließen. Beispielsweise kann die Spitze der Insertionsnadel mit der ersten Vorschubbewegung aus dem Insertionsgerät herausgeschoben werden, wobei eine anschließende Rückzugsbewegung endet, bevor die Spitze der Insertionsnadel wieder in das Innere des Insertionsgeräts eintritt.

Bevorzugt weicht die Orientierung der Insertionsnadel am Ende eines Einstiches um mindestens 20°, besonders bevorzugt um mindestens 40°, insbesondere um mindestens 70° von der Orientierung der Insertionsnadel bei Beginn des Einstichs und damit auch von der anfänglichen Vorschubrichtung ab. Im Allgemeinen gilt, dass sich der Vorteil einer größeren nutzbaren Sensorlänge bei schmerzarmem Einstich und geringer Gefahr einer Verletzung von tiefer liegendem Muskelgewebe in umso größerem Maße nutzen lässt, je weniger die Orientierung der Insertionsnadel beim Auftreffen auf die Hautoberfläche von einer Senkrechten zur Hautoberfläche abweicht und je mehr die Orientierung der Insertionsnadel am Ende der Einstichbewegung von der Senkrechten abweicht. Medizinisch gesehen ist also ein senkrechtes Auftreffen der Insertionsnadel auf die Haut und eine waagerechte Orientierung des Sensors unter der Haut optimal. Allerdings ist die Bewegungssteuerung der Insertionsnadel im Allgemeinen umso aufwändiger, je mehr die Vorschubrichtung bei einem Einstich geändert werden soll. Eine geeignete Bewegungssteuerung kann beispielsweise durch einen Stechmechanismus mit einer Kulissensteuerung erreicht werden. Eine weitere Möglichkeit sind Kuppelgetriebe, beispielsweise ein Viergelenk.

Bei einem erfindungsgemäßen Verfahren zum Steuern der Stechbewegung einer von einer Insertionsvorrichtung bewegten Nadel mittels einer kulissensteuerung wobei die Nadel mit der Stechbewegung nicht in einen lebenden menschlichen oder tierischen Körper gestochen wird, wird die Nadel zu Beginn der Stechbewegung linear in einer Vorschubrichtung bewegt, die mit der Längsrichtung der Nadel übereinstimmt. Während des Stichs wird die Vorschubrichtung der Nadel geändert so dass die Nadel am Ende des Stichs schräg oder senkrecht zu der anfänglichen Vorschubrichtung ausgerichtet ist. Die Orientierungen der Nadel zu Beginn der Stechbewegung und am Ende der Stechbewegung sind also schräg oder senkrecht zueinander.

Nach einer ersten Vorschubbewegung wird erfindungsgemäß die Nadel um einen Teil des dabei bewirkten Vorschubs zurückgezogen und später bei einer weiteren Vorschubbewegung schräg oder senkrecht zu der Richtung der ersten Vorschubbewegung vorgeschoben. Die Rückzugsbewegung kann ebenso wie die erste Vorschubbewegung eine lineare Bewegung sein.

Eine geeignete Bewegungssteuerung kann beispielsweise mit einer Kulissensteuerung realisiert werden, deren Steuerkurve einen ersten, geradlinigen, Abschnitt für die erste Vorschubbewegung aufweist. An den ersten Abschnitt schließt ein zweiter Abschnitt an, mit dem die Vorschubrichtung geändert wird. Der zweite Abschnitt ist bevorzugt bogenförmig, kann an sich jedoch auch geradlinig, schräg oder senkrecht zu dem ersten Abschnitt verlaufen. Der zweite Abschnitt kann an das Ende des ersten Abschnitts anschließen. Um eine Rückzugsbewegung zu bewirken, kann der zweite Abschnitt der Steuerkurve aber auch zwischen Anfang und Ende des ersten Abschnitts beginnen. Ein Steuerkurvenreiter fährt dann zunächst vom Anfang des ersten Abschnitts zum Ende des ersten Abschnitts und bewegt sich dabei am Anfang des zweiten Abschnitts vorbei. Anschließend wird ein Teil des ersten Abschnitts in umgekehrter Richtung abgefahren bis der Anfang des zweiten Abschnitts erreicht ist. An den zweiten Abschnitt der Steuerkurve schließt bevorzugt ein dritter Abschnitt an, der eine zweite Vorschubbewegung bewirkt. Bevorzugt ist der dritte Abschnitt geradlinig ausgebildet.

Mit dem beschriebenen Verfahren zum Steuern der Stechbewegung einer von einer Insertionsvorrichtung bewegten Nadel kann vorteilhaft erreicht werden, dass die Nadel mit einem steilen Winkel oder sogar senkrecht auf die Haut auftrifft und nach dem Durchstoßen der Haut die Vorschubrichtung der Nadel geändert wird, so dass ein Sensor trotz einer kleinen Eindringtiefe auf großer Länge von Fettgewebe umgeben ist. Eine entsprechend eingerichtete Insertionsvorrichtung führt das erfindungsgemäße Verfahren zum Steuern der Stechbewegung naturgemäß aus, sobald ein Stich ausgelöst wird, auch wenn die Insertionsvorrichtung nicht an die Haut eines Patienten angesetzt ist, so dass die Nadel dann nicht in einen Patienten eindringt.

Weitere Einzelheiten und Vorteile der Erfindung werden an einem Ausführungsbeispiel unter Bezugnahme auf die beigefügten Zeichnungen erläutert. Es zeigen:
Figur 1 eine schematische Darstellung eines Insertionssystems; und
Figur 2 eine schematische Darstellung der Bewegungsbahn einer Insertionsnadel.

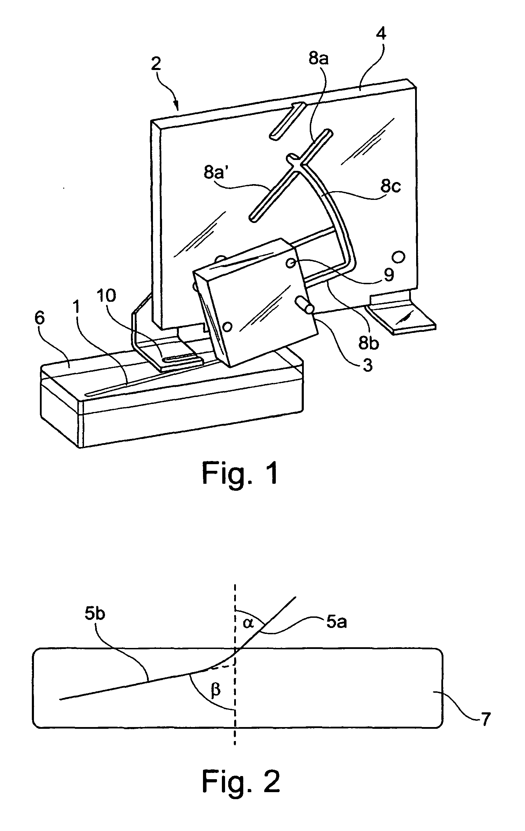

In Figur 1 ist schematisch ein Insertionssystem zum Insertieren eines Sensors in Fettgewebe des Bauchs dargestellt. Das Insertionssystem besteht aus einer einen Sensor enthaltenden Insertionsnadel 1 und einem Insertionsgerät 2. Das Insertionsgerät 2 ist in Figur 1 ohne Gehäuse, Bedienungselemente und ähnliche Teile dargestellt, die zum Verständnis der Stichbewegung und dazugehörenden Bewegungssteuerung nicht erforderlich sind.

Das Insertionsgerät 2 hat einen Halter 3 für die geradlinige Insertionsnadel 1 und einen Stechmechanismus 4, um den Halter 3 zusammen mit einer von ihm gehaltenen Nadel 1 für einen Stich zu bewegen. Der Stechmechanismus kann einen elektrischen Antrieb haben oder beispielsweise von einem Benutzer aufgebrachte Muskelkraft in eine Stechbewegung umsetzen, wie dies beispielsweise aus der WO 2010/040448 A1 oder der DE 10 2004 059 491 A1 bekannt ist.

Die Insertionsnadel 1 kann beispielsweise einfach eine geschlitzte Kanüle sein, die direkt von dem Halter 3 des Insertionsgeräts gehalten ist. Möglich ist es auch, dass die Insertionsnadel einen Kunststoffkörper aufweist, in dem eine Kanüle steckt. In einem solchen Fall genügt es, wenn der Halter 3 des Insertionsgeräts den Kunststoffkörper der Insertionsnadel hält. Allgemein gesagt, kann der Halter 3 des Insertionsgeräts die Insertionsnadel 1 unmittelbar oder mittelbar halten.

Der Stechmechanismus 4 des in Figur 1 schematisch dargestellten Insertionssystems bewirkt bei einem Stich die in Figur 2 dargestellte Bewegungsbahn 5a, 5b. In Figur 2 ist dabei die Bahn der Spitze einer Insertionsnadel 1 beim Auftreffen auf eine Hautoberfläche 6 und daran anschließend im darunter liegenden Fettgewebe 7 schematisch dargestellt. Die Bewegungsbahn enthält einen geradlinig verlaufenden Anfangsabschnitt 5a und einen geradlinig verlaufenden Endabschnitt 5b, der schräg zu dem Anfangsabschnitt 5a verläuft.

Figur 2 zeigt also, dass der Stechmechanismus des in Figur 1 schematisch dargestellten Insertionsgeräts den Halter und damit auch eine von ihm gehaltene Insertionsnadel bei einem Stich zunächst in einer ersten Vorschubrichtung bewegt und danach während des Stichs die Vorschubrichtung ändert, so dass eine von dem Halter gehaltene Insertionsnadel am Ende des Stichs schräg zu der ersten Vorschubrichtung ausgerichtet ist. Die von dem Halter und damit auch die von der Insertionsnadel bei einem Stich ausgeführte Bewegung ist in einem ersten Bewegungsabschnitt 5a eine lineare Bewegung in der ersten Vorschubrichtung und in einem weiteren Bewegungsabschnitt 5b eine lineare Bewegung in einer zweiten Vorschubrichtung, die schräg zu der ersten Vorschubrichtung orientiert ist. Zwischen den beiden linearen Bewegungsabschnitten 5a, 5b führt der Halter 3 in dem dargestellten Ausführungsbeispiel eine bogenförmige Bewegung durch.

Die Insertionsnadel 1 schließt deshalb beim Auftreffen auf die Hautoberfläche 6 eines Patienten mit einer Senkrechten zur Hautoberfläche einen ersten Winkel α ein. Nach dem Durchstoßen der Haut ändert der Stechmechanismus die Vorschubrichtung der Insertionsnadel, so dass die Insertionsnadel 1 am Ende der Einstichbewegung mit der Senkrechten zur Hautoberfläche einen zweiten Winkel β einschließt, der größer als der erste Winkel α ist. Durch ein steiles Auftreffen wird eine Lateralverschiebung der Haut 6 um die Einstichstelle vermieden, während der zweite lineare Bewegungsabschnitt für eine gute Verankerung des Sensors im Fettgewebe sorgt. Die Länge des Sensors kann bei der in Figur 2 dargestellten Bewegungsbahn fasst beliebig lang gewählt werden. Der erste Winkel α beträgt bevorzugt zwischen 0° und 45°. Der zweite Winkel β beträgt bevorzugt 45° bis 90°. Die Orientierung der Insertionsnadel 1 am Ende eines Einstichs weicht bei dem dargestellten Ausführungsbeispiel um mehr als 30° von der Orientierung der Insertionsnadel 1 bei Beginn des Einstichs ab.

Die in Figur 2 dargestellte Bewegungsbahn wird bei dem dargestellten Ausführungsbeispiel mit einer Kulissensteuerung erzeugt, also einer Steuerkurve, die von einem Steuerkurvenreiter 9, beispielsweise einem mit der Halterung 3 verbundenen Zapfen, abgefahren wird.

Die Steuerkulisse enthält bei dem dargestellten Ausführungsbeispiel eine Steuerkurve, die zwei lineare Abschnitte 8a, 8b aufweist, die schräg zueinander verlaufen und durch einen bogenförmigen Abschnitt 8c verbunden sind. Jeder der beiden linearen Abschnitte 8a, 8b der Steuerkurve bewirkt einen linearen Bewegungsabschnitt der Stichbewegung. Der gebogene Abschnitt der Steuerkurve 8c bewirkt eine Änderung der Vorschubrichtung, so dass der zweite lineare Bewegungsabschnitt 5b schräg zu dem ersten linearen Bewegungsabschnitt 5a verläuft.

Bei dem dargestellten Ausführungsbeispiel kann die Insertionsnadel 1 nach einem ersten linearen Bewegungsabschnitt 5a zunächst wieder etwas zurückgezogen werden, bevor die Orientierung der Nadel durch den Steuerkurvenabschnitt 8c geändert wird. Der gebogene Steuerkurvenabschnitt 8c schließt deshalb nicht an das Ende des linearen Steuerkurvenabschnitt 8a an. Der in Figur 1 dargestellte Steuerkurvenabschnitt 8a' kann für eine Vorschub- und anschließende Rückführbewegung der Nadel 1 genutzt werden. Bei dem dargestellten Ausführungsbeispiel führt die Nadel zur Änderung ihrer Orientierung einer Schwenkbewegung aus, während die Nadelspitze aus dem Insertionsgerät hinaus ragt, also in Fettgewebe steckt.

Das in Figur 1 schematisch dargestellte Insertionsgerät hat an seiner Unterseite eine Austrittsöffnung 10 für die Insertionsnadel 1. Die Austrittsöffnung 10 ist bei dem dargestellten Ausführungsbeispiel ein Schlitz. Indem die Vorschubrichtung der Insertionsnadel durch eine Schwenkbewegung geändert wird, wie dies bei dem dargestellten Ausführungsbeispiel der Fall ist, lassen sich die nötige Beweglichkeit der Insertionsnadel 1 nach Durchstechen der Hautoberfläche realisieren und die Hautoberfläche günstig fixieren, um das Durchstoßen zu erleichtern.

Der Stechmechanismus ändert die Vorschubrichtung der Insertionsnadel, nachdem die Spitze der Insertionsnadel 1 aus der Austrittsöffnung 10 des Stechgeräts ausgetreten ist, also die Hautoberfläche 6 durchstoßen ist. An sich kann die Vorschubrichtung der Insertionsnadel 1 bereits vor dem Auftreffen auf die Hautoberfläche etwas geändert werden. Bevorzugt ändert der Stechmechanismus die Vorschubrichtung der Insertionsnadel aber erst, nachdem die Spitze der Insertionsnadel 1 aus der Austrittsöffnung 10 des Stechgeräts ausgetreten ist. Um die Vorteile einer Änderung der Vorschubrichtung nutzen zu können, kommt es auf die Änderung der Vorschubrichtung nach Durchstechen der Haut 6 an. Änderungen der Vorschubrichtung vor dem Durchstechen der Haut 6 sind zwar nicht schädlich, bringen jedoch auch keinen Vorteil und verkomplizieren in der Regel lediglich die Steuerung.

Der Rand der Austrittsöffnung 10 definiert eine Fläche. Beim Austreten der Spitze der Insertionsnadel 1 aus der Austrittsöffnung 10 schließt die Insertionsnadel mit einer Senkrechten zu dieser Fläche den ersten Winkel α ein. Die Senkrechte zu der durch den Rand der Austrittsöffnung 10 definierten Fläche stimmt bei einem Stich mit der Senkrechten zur Hautoberfläche überein. Der Stechmechanismus ändert nach dem Austritt der Nadelspitze aus der Öffnung 10 die Vorschubrichtung der Insertionsnadel 1, so dass die Insertionsnadel 1 am Ende der Einstichbewegung mit der Senkrechten zu der durch den Rand der Austrittsöffnung 10 definierten Fläche den zweiten Winkel β einschließt.

### Bezugszeichen

- 1: Insertionsnadel
- 2: Insertionsgerät
- 3: Halter
- 4: Stechmechanismus
- 5a: Anfangsabschnitt der Bewegungsbahn
- 5b: Endabschnitt der Bewegungsbahn
- 6: Hautoberfläche
- 7: Fettgewebe
- 8a: Steuerkurvenabschnitt
- 8b: Steuerkurvenabschnitt
- 8c: Steuerkurvenabschnitt
- 9: Steuerkurvenreiter
- 10: Austrittsöffnung

- α: erster Winkel
- β: zweiter Winkel

## Patentansprüche

1. Insertionssystem zum Insertieren eines Sensors oder einer Kanüle in Fettgewebe (7), mit
einem Insertionsgerät (2) und einer einen Sensor oder einer Kanüle enthaltenden Insertionsnadel (1),
wobei das Insertionsgerät (2) einen Halter (3) für die Insertionsnadel (1) und einen Stechmechanismus (4), um den Halter (3) mit einer von ihm gehaltenen Nadel (1) für einen Stich linear in einer Vorschubrichtung zu bewegen, aufweist, wobei der Stechmechanismus (4) während des Stichs die Vorschubrichtung ändert, so dass eine von dem Halter (3) gehaltene Insertionsnadel (1) am Ende des Stichs schräg oder senkrecht zu der anfänglichen Vorschubrichtung ausgerichtet ist, und
wobei der Halter (3) bei einem Stich eine Bewegung durchführt, die einen Bogen enthält,
wobei der Stechmechanismus (4) eine Kulissensteuerung enthält,
**dadurch gekennzeichnet, dass** der Stechmechanismus (4) den Halter (3) nach einer ersten Vorschubbewegung (5a) zurückzieht und später eine zweite Vorschubbewegung (5b) bewirkt,
der Stechmechanismus (4) die Vorschubrichtung der Insertionsnadel (1) ändert, nachdem die Spitze der Insertionsnadel (1) aus einer Austrittsöffnung (10) des Stechgeräts ausgetreten ist,
wobei die Insertionsnadel (1) beim Austreten ihrer Spitze aus der Austrittsöffnung (10) während der ersten Vorschubbewung (5a) mit einer Senkrechten zu einer durch den Rand der Öffnung definierten Fläche einen ersten Winkel (α) einschließt, der Stechmechanismus (4) danach die Vorschubrichtung der Insertionsnadel (1) ändert, so dass die Insertionsnadel (1) Während der Zweiten Vorschubbewegung (5b) und am Ende der Einstichbewegung mit der Senkrechten einen zweiten Winkel (β) einschließt, wobei der zweite Winkel (β) größer als der erste Winkel (α) ist.

2. Insertionssystem nach einem der vorstehenden Ansprüche, **dadurch gekennzeichnet, dass** der Halter (3) bei einem Stich eine Bewegung ausführt, die einen Bewegungsabschnitt (5a) mit einer linearen Vorschubbewegung in einer ersten Vorschubrichtung und einen späteren Bewegungsabschnitt (5b) mit einer linearen Bewegung in einer zweiten Vorschubrichtung, die schräg oder senkrecht zu der ersten Vorschubrichtung orientiert ist, enthält.

3. Insertionssystem nach einem der vorstehenden Ansprüche, **dadurch gekennzeichnet, dass** der Stechmechanismus (4) die Vorschubrichtung der Insertionsnadel (1) erst ändert, nachdem die Spitze der Insertionsnadel (1) aus einer Austrittsöffnung (10) des Stechgeräts ausgetreten ist.

4. Insertionssystem nach einem der vorstehenden Ansprüche, **dadurch gekennzeichnet, dass** die Austrittsöffnung (10) ein Schlitz ist.

5. Insertionssystem nach einem der vorstehenden Ansprüche, **dadurch gekennzeichnet, dass** die Insertionsnadel (1) bei Auftreffen auf die Hautoberfläche (6) eines Patienten mit einer Senkrechten zur Hautoberfläche (6) einen ersten Winkel (α) einschließt, der Stechmechanismus (4) nach dem Durchstoßen der Haut die Vorschubrichtung der Insertionsnadel (1) ändert, so dass die Insertionsnadel (1) am Ende der Einstichbewegung mit der Senkrechten zur Hautoberfläche (6) einen zweiten Winkel (β) einschließt, wobei der zweite Winkel (β) größer als der erste Winkel (α) ist.

6. lnsertionssystem nach einem der vorstehenden Ansprüche, **dadurch gekennzeichnet, dass** der erste Winkel (α) 0° bis 45° beträgt und der zweite Winkel (β) 45° bis 90° beträgt.

7. Insertionssystem nach einem der vorstehenden Ansprüche, **dadurch gekennzeichnet, dass** die Orientierung der Insertionsnadel (1) am Ende eines Einstichs um mindestens 20°, vorzugsweise mindestens 40°, insbesondere mindestens 70°, von der Orientierung der Insertionsnadel (1) bei Beginn eines Einstichs abweicht.

8. Verfahren zum Steuern der Stechbewegung einer von einem insertions system gemäß Anspruch 1 bewegten Nadel (1) mittels einer Kulissensteuerung,
wobei die Nadel (1) mit der Stechbewegung nicht in einen lebenden menschlichen oder tierischen Körper gestochen wird, und
wobei die Nadel (1) zu Beginn eine Stichs geradlinig bewegt und dann während des Stichs die Vorschubrichtung der Nadel (1) geändert wird, so dass die Nadel (1) am Ende des Stichs schräg oder senkrecht zu der anfänglichen Vorschubrichtung ausgerichtet ist, **dadurch gekennzeichnet, dass** die Nadel (1) nach einer ersten Vorschubbewegung um einen Teil des dabei bewirkten Vorschubs zurückgezogen wird und später bei einer weiteren Vorschubbewegung schräg oder senkrecht zu der Richtung der ersten Vorschubbewegung vorgeschoben wird.

9. Verfahren nach Anspruch 8, **dadurch gekennzeichnet, dass** die Vorschubrichtung durch eine Schwenkbewegung der Nadel (1) geändert wird.

## Claims

1. An insertion system for inserting a sensor or a cannula into fatty tissue (7), comprising
an insertion device (2) and an insertion needle (1) containing a sensor or a cannula,
the insertion device (2) having a holder (3) for the insertion needle (1) and a piercing mechanism (4) for moving the holder (3), together with a needle (1) held by the same, for a puncture linearly in a feed direction,
wherein the piercing mechanism (1) changes the feed direction during the puncture so that, at the end of the puncture, an insertion needle (1) held by the holder (3) is oriented obliquely or perpendicular to the initial feed direction, and
wherein during a puncture the holder (3) carries out a movement that contains a curve,
wherein the piercing mechanism (4) contains a cam control,
**characterized in that** the piercing mechanism (4) pulls the holder (3) back after a first feed movement (5a) and later causes a second feed movement (5b),
the piercing mechanism (4) changes the feed direction of the insertion needle (1) after the tip of the insertion needle (1) has exited the exit opening (10) of the piercing device,
wherein during the first feed movement the insertion needle (1) forms a first angle (α) with a perpendicular in relation to an area defined by the edge of the opening when the tip exits the exit opening (10), and the piercing mechanism (4) then changes the feed direction of the insertion needle (1) so that, during the second feed movement and at the end of the piercing movement, the insertion needle (1) forms a second angle (β) with the perpendicular, the second angle (β) being greater than the first angle (α).

2. An insertion system according to any one of the preceding claims, **characterized in that** during a puncture the holder (3) carries out a movement that contains a movement section (5a) with a linear feed movement in a first feed direction and a later movement section (5b) with a linear movement in a second feed direction, which is oriented obliquely or perpendicularly to the first feed direction.

3. The insertion system according to any one of the preceding claims, **characterized in that** the piercing mechanism (4) does changes the feed direction of the insertion needle (1) only after the tip of the insertion needle (1) has exited an exit opening (10) of the piercing device.

4. An insertion system according to any one of the preceding claims, **characterized in that** the exit opening (10) is a slot.

5. An insertion system according to any one of the preceding claims, **characterized in that**, upon impingement on the skin surface (6) of a patient, the insertion needle (1) forms a first angle (α) with a perpendicular in relation to a skin surface (6), and after the skin has been penetrated, the piercing mechanism (4) changes the feed direction of the insertion needle (1) so that, at the end of the piercing movement, the insertion needle (1) forms a second angle (β) with the perpendicular in relation to the skin surface (6), the second angle (β) being greater than the first angle (α).

6. The insertion system according to any one of the preceding claims, **characterized in that** the first angle (α) is 0° to 45° and the second angle (β) is 45° to 90°.

7. An insertion system according to any one of the preceding claims, **characterized in that** the orientation of the insertion needle (1) at the end of a puncture deviates from the orientation of the insertion needle (1) at the beginning of a puncture by at least 20°, preferably by at least 40°, and more particularly by at least 70°.

8. A method for controlling the piercing movement of a needle (1) moved by an insertion system according to claim 1 by means of a cam control,
wherein the needle (1) is not introduced into a living body of a human or animal, and
wherein at the beginning of a puncture, the needle (1) is moved in a rectilinear fashion and then, during the puncture, the feed direction of the needle (1) is changed so that at the end of the puncture the needle (1) is oriented obliquely or perpendicularly to the initial feed direction**characterized in that**, after a first feed movement, the needle (1) is pulled back by a portion of the advancement that was caused, and later, during a further feed movement, it is pushed forward obliquely or perpendicularly to the direction of the fist feed movement.

9. The method according to claim 8, **characterized in that** the feed direction is changed by a pivot movement of the needle (1).

## Revendications

1. Système d'insertion destiné à insérer un capteur ou une canule dans le tissu adipeux (7), comprenant
un dispositif d'insertion (2) et une aiguille d'insertion (1) contenant un capteur ou une canule,
dans lequel le dispositif d'insertion (2) comporte un support (3) destiné à l'aiguille d'insertion (1) et un mécanisme de piqûre (4) permettant de déplacer linéairement le support (3), y compris une aiguille (1) portée par celui-ci, dans le sens de l'avance en vue d'une piqûre,
dans lequel le mécanisme de piqûre (4) modifie le sens de l'avance pendant la piqûre, de sorte qu'une aiguille d'insertion (1) portée par le support (3) soit orientée obliquement ou perpendiculairement au sens initial de l'avance après la piqûre, et
dans lequel le support (3), lors d'une piqûre, effectue un mouvement comprenant une courbe,
dans lequel le mécanisme de piqûre (4) comprend une commande à coulisse,
**caractérisé en ce que** le mécanisme de piqûre (4) fait reculer le support (3) après un premier mouvement d'avance (5a) et déclenche ultérieurement un deuxième mouvement d'avance (5b),
le mécanisme de piqûre (4) modifie le sens de l'avance de l'aiguille d'insertion (1) une fois que la pointe de l'aiguille d'insertion (1) est sortie d'un orifice de sortie (10) du dispositif de piqûre,
laquelle aiguille d'insertion (1), au moment où sa pointe sort de l'orifice de sortie (10), renferme pendant le premier mouvement d'avance (5a) un premier angle (α) avec une verticale par rapport à une surface définie par le bord dudit orifice, puis le mécanisme de piqûre (4) modifie le sens de l'avance de l'aiguille d'insertion (1), de sorte que l'aiguille d'insertion (1) renferme pendant le deuxième mouvement d'avance (5b) et à la fin du mouvement de piqûre un deuxième angle (β) avec la verticale, le deuxième angle (13) étant plus grand que le premier angle (α).

2. Système d'insertion selon l'une des revendications précédentes, **caractérisé en ce que** le support (3), lors d'une piqûre, effectue un mouvement comprenant un premier tronçon (5a) avec un mouvement d'avance linéaire dans un premier sens de l'avance, et un tronçon (5b) venant après, avec un mouvement linéaire dans un deuxième sens de l'avance, orienté obliquement ou perpendiculairement au premier sens de l'avance.

3. Système d'insertion selon l'une des revendications précédentes, **caractérisé en ce que** le mécanisme de piqûre (4) ne modifie le sens de l'avance de l'aiguille d'insertion (1) qu'après que la pointe de l'aiguille d'insertion (1) est sortie d'un orifice de sortie (10) du dispositif de piqûre.

4. Système d'insertion selon l'une des revendications précédentes, **caractérisé en ce que** l'orifice de sortie (10) est une fente.

5. Système d'insertion selon l'une des revendications précédentes, **caractérisé en ce que** l'aiguille d'insertion (1), lorsqu'elle rencontre la surface de la peau (6) d'un patient, renferme un premier angle (α) avec une verticale par rapport à la surface de la peau (6), puis le mécanisme de piqûre (4), après avoir percé la peau, modifie le sens de l'avance de l'aiguille d'insertion (1), de sorte que l'aiguille d'insertion (1) renferme à la fin du mouvement de piqûre un deuxième angle (β) avec la verticale par rapport à la surface de la peau (6), le deuxième angle (β) étant plus grand que le premier angle (α).

6. Système d'insertion selon l'une des revendications précédentes, **caractérisé en ce que** le premier angle (α) est compris entre 0° et 45° et le deuxième angle (β) entre 45° et 90°.

7. Système d'insertion selon l'une des revendications précédentes, **caractérisé en ce que** l'orientation de l'aiguille d'insertion (1) à la fin d'une piqûre diffère d'au moins 20°, de préférence d'au moins 40°, en particulier d'au moins 70°, de l'orientation de l'aiguille d'insertion (1) au début d'une piqûre.

8. Procédé pour commander le mouvement de piqûre d'une aiguille (1) mue par un système d'insertion selon la revendication 1 au moyen d'une commande à coulisse,
dans lequel l'aiguille (1) ne pénètre pas dans le corps humain ou animal vivant lors du mouvement de piqûre, et
dans lequel l'aiguille (1), au début d'une piqûre, est mue en ligne droite, puis pendant la piqûre le sens de l'avance de l'aiguille (1) est modifié, de sorte que l'aiguille (1) est orienté à la fin de la piqûre obliquement ou perpendiculairement au sens initial de l'avance, **caractérisé en ce que** l'aiguille (1), après un premier mouvement d'avance, recule d'une partie de l'avance alors engendrée et avance ultérieurement, lors d'un autre mouvement d'avance, obliquement ou perpendiculairement au sens du premier mouvement d'avance.

9. Procédé selon la revendication 8, **caractérisé en ce que** le sens de l'avance est modifié par un pivotement de l'aiguille (1).
